# EUROPEAN PATENT APPLICATION

(11) **EP 2 098 308 A2**
(43) Date of publication of application: **09.09.2009**
(21) Application number: 09154550.9
(22) Date of filing: 06.03.2009
(51) Int. Cl.: B09B 3/00, B03B 9/06, B01J 3/04

(54) **Methods and Systems for Solid Waste Processing**

(30) Priority: 06.03.2008 US 34337 P
(71) Applicant: GENERAL KINEMATICS CORPORATION, Crystal Lake, IL 60014 (US)
(72) Inventor: BRITTON, Dan, Barrington, IL, 60010 (US)
(74) Representative: Bardehle, Heinz

(57) **Abstract**

A method of processing solid waste material includes storing solid waste material in a storage conveyor (120), the conveyor including a trough (140) with a deck (142) and opposing side walls (144) disposed to either side of the deck (142), a plurality of resilient members (146) supporting the trough (140) above a surface, and a vibration generator (154) coupled to the trough (140) to move solid waste material stored in the trough (140) along the deck (142) to an outlet (156). The method also includes activating the vibration generator (154) to move the solid waste material along the deck (142) to the outlet, receiving the solid waste material in a container (170), and supplying steam to the container to heat the solid waste material received therein to generate treated solid waste material. A system (100) for carrying out the process is also provided.

## Description

### Background

This patent is directed to methods and systems for solid waste processing, and, in particular, to methods and systems for solid waste processing utilizing vibratory equipment with mixed solid waste material.

### Summary

According to an aspect of the present disclosure, a vibratory system for processing solid waste material includes a storage conveyor including a trough with a deck and opposing side walls disposed to either side of the deck, a plurality of resilient members supporting the trough above a surface, and a vibration generator coupled to the trough to move solid waste material disposed in the trough along the deck to an outlet. The system also includes a heating unit including a container with an opening to receive solid waste material from the outlet of the storage conveyor, the opening being in communication with an interior chamber, a closure moveable relative to the opening to limit passage of solid waste material through the opening, and a source of steam coupled to the interior chamber to provide steam to the interior chamber to heat solid waste material disposed therein.

According to another aspect of the present disclosure, a method of processing solid waste material includes storing solid waste material in a storage conveyor, the conveyor including a trough with a deck and opposing side walls disposed to either side of the deck, a plurality of resilient members supporting the trough above a surface, and a vibration generator coupled to the trough to move solid waste material stored in the trough along the deck to an outlet. The method also includes activating the vibration generator to move the solid waste material along the deck to the outlet, receiving the solid waste material in a container, and supplying steam to the container to heat the solid waste material received therein to generate treated solid waste material.

### Brief Description of the Drawings

Figs. 1A and 1B are a plan view of a system for solid waste processing according to the present disclosure;
Fig. 2A is a side elevation view of a combination of a storage conveyor, a feed conveyor and a heating unit, with the feed conveyor in a first position;
Fig. 2B is a side elevation view of the combination of storage conveyor, feed conveyor and heating unit of Fig. 2A, with the feed conveyor in a second position;
Fig. 3 is a side elevation view of a combination of transfer conveyors and screening conveyors;
Fig. 4 is a side elevation view of a combination of transfer conveyors and presses;
Fig. 5A is a side elevation view of a press for use in the sub-system of Fig. 4;
Fig. 5B is a cross-sectional view of the press of Fig. 5A; and
Fig. 6 is a side elevation view of a combination of the presses from Fig. 4, dryers and associated transfer conveyors;
Fig. 7 is a side elevation view of a first combination of transfer conveyors and picking stations associated with the screening conveyors of Fig. 3; and
Fig. 8 is a side elevation view of a second combination of transfer conveyors and picking stations associated with the screening conveyors of Fig. 3.

### Detailed Description of Various Embodiments

Although the following text sets forth a detailed description of different embodiments of the invention, it should be understood that the legal scope of the invention is defined by the words of the claims set forth at the end of this patent. The detailed description is to be construed as exemplary only and does not describe every possible embodiment of the invention since describing every possible embodiment would be impractical, if not impossible. Numerous alternative embodiments could be implemented, using either current technology or technology developed after the filing date of this patent, which would still fall within the scope of the claims defining the invention.

It should also be understood that, unless a term is expressly defined in this patent using the sentence "As used herein, the term '____ ' is hereby defined to mean..." or a similar sentence, there is no intent to limit the meaning of that term, either expressly or by implication, beyond its plain or ordinary meaning, and such term should not be interpreted to be limited in scope based on any statement made in any section of this patent (other than the language of the claims). To the extent that any term recited in the claims at the end of this patent is referred to in this patent in a manner consistent with a single meaning, that is done for sake of clarity only so as to not confuse the reader, and it is not intended that such claim term be limited, by implication or otherwise, to that single meaning. Finally, unless a claim element is defined by reciting the word "means" and a function without the recital of any structure, it is not intended that the scope of any claim element be interpreted based on the application of 35 U.S.C. §112, sixth paragraph.

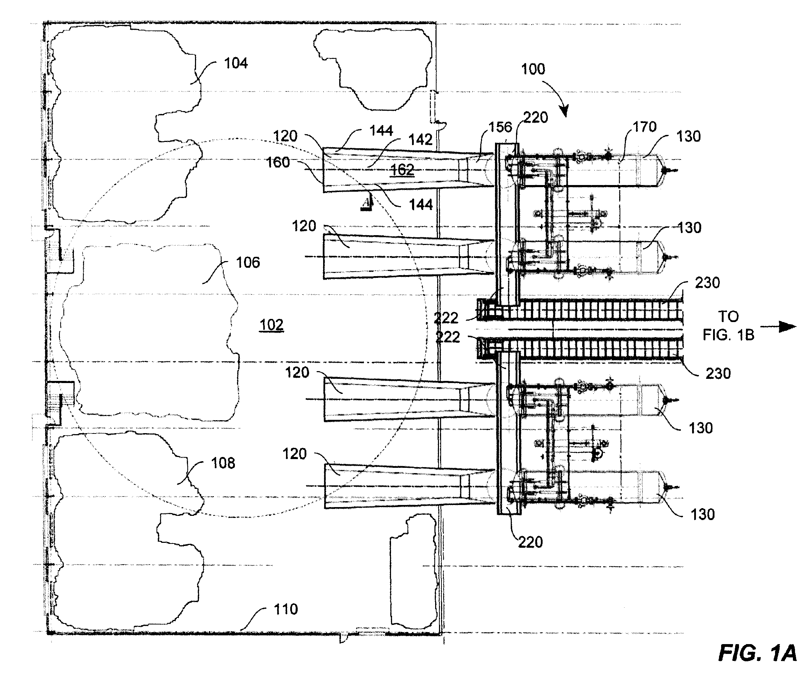

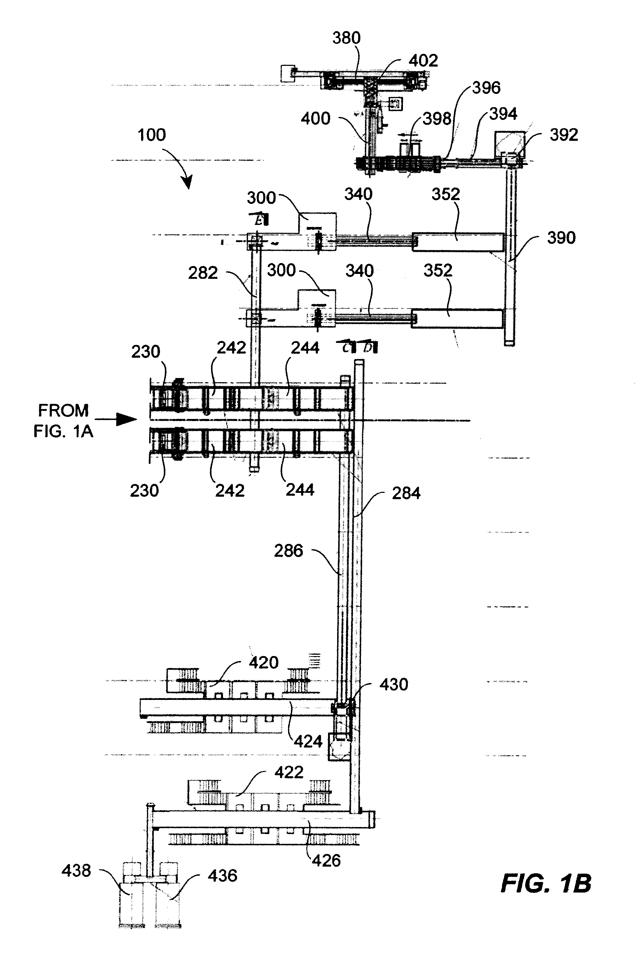

Referring first to Figs. 1A and 1B, a system 100 for processing solid waste material is illustrated. The waste material received by the system 100 may include a mix of materials, such as newspapers, boxes, bottles, cans, food wastes, etc., as may be found in solid municipal waste. Thus, the solid waste material may include a variety of material types, such as paper, cardstock, cardboard, glass, plastic, metal, biomaterials, etc. The solid waste material may be sorted before it is loaded into the system 100, but the system 100 is designed to treat unsorted solid waste material as well.

Starting then at the left-hand side of Fig. 1A, there is a space 102 in which a plurality of piles 104, 106, 108 may be disposed. These piles 104, 106, 108 are composed of solid waste materials, some piles being sorted before their arrival in the space 102 and other piles being unsorted. The space 102 is preferably an enclosed space, defined by a structure 110 so as to prevent the solid waste materials from being carried out of the space 102 by the action of wind and water, for example. An enclosed space 102 also limits the access that animals, in particular larger animals, have to the solid waste material.

A transport device (not shown) may be disposed in the space 102 to load the solid waste materials of the piles 104, 106, 108 into the system 100. Such a transport device may take the form of a vehicle, such as a front loader, for example. Alternatively, the transport device may be in the form of an overhead crane fitted with a claw, a bucket or the like. Still further, the transport device may include a conveyor on which workers manually dispose the solid waste material for its transit along the conveyor into the system 100.

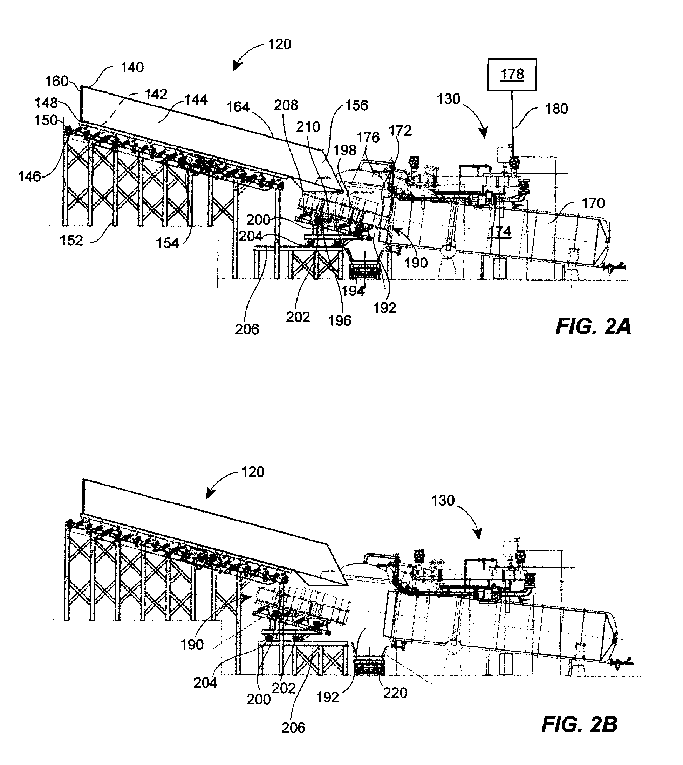

The system 100 includes at least one storage conveyor 120. Four storage conveyors 120 are illustrated in Fig. 1A. The storage conveyors 120 are used to store the solid waste material 120 prior to its receipt in one of a plurality of heating units 130. Each conveyor 120 may hold a sufficient amount of solid waste material to fill one of the heating units 130 according to the illustrated embodiment. However, it will be recognized that the conveyor 120 may be sized to store either more or less solid waste material than is required to fill one of the heating units 130. Referring now to Figs. 1A, 2A and 2B, the storage conveyor 120 may include a trough 140. The trough 140 has a deck 142 and opposing side walls 144 disposed to either side of the deck 142. A plurality of resilient members 146 supports the trough 140 above a surface. As illustrated in Fig. 2A and 2B, the resilient members 146 (which may be coil springs) are attached at one end 148 to the trough 140 and at a second end 150 to a frame 152. A vibration generator 154 is coupled to the trough 140 to move solid waste material disposed in the trough 140 along the deck 142 to an outlet 156.

According to the illustrated embodiment, the frame 152 is required to provide a certain elevation for the solid waste material relative to other elements of the system 100, such as the heating unit 130. The size of the frame 152 is also dictated, in part, by the relative elevations of the surfaces on which the different elements of the system 100 are supported. It will be recognized that variations in elevation of the elements and surfaces will influence the construction of the system 100 in general, and of the frame 152 in particular.

It will also be recognized that considerable variation may occur relative to the suspension of the trough 140 relative to the surface on which it is supported, and in regard to the vibration generator 154. That is, the resilient members 146 are typically paired with rigid linkages to support the trough 140, as illustrated. The vibration generator 154 may be a two-mass system or may be a one-mass (or brute force) system. The vibration generator 154 may be coupled to the trough 140, or may be mounted on a counterpoise which may be attached to the trough 140. All of these elements may vary according the demands placed on the system 100.

Importantly, the storage conveyor 120 provides the afore-mentioned trough 140 with continuous deck 142 flanked by opposing side walls 144. The trough 140 may even include an end wall 160 at the end of the deck 142 opposite the outlet 156. The deck 142, side walls 144 and end wall 160 thus define a space 162 (see Fig. 1A) into which and from which passage is restricted. The solid waste material is disposed into the space 162 through the open top 164 (see Figs. 1A and 2A), and exits the space 162 through the outlet 156.

This is to be contrasted with a belt conveyor, wherein clearance typically exists between the belt and any side walls that may be provided to guide the solid waste material along the conveyor. Solid waste materials may pass through the clearance between the belt and the side walls, if any, and become disposed in a space that may be extremely difficult to clean regularly and/or completely. Where the solid waste material includes food and other biomaterials, the material trapped in this space may provide encourage an increase in the number of insects, rodents and the like. This may result in an extremely unhealthy environment in which to work.

Thus, it will be recognized that by providing a vibratory conveyor 120, wherein the solid waste material is contained in the enclosed space 162 from which passage is restricted, the problems presented through the use of a belt or similar type of conveyor may be reduced or eliminated. Solid waste material does not end up being lost into the working gear of the conveyor 120, as the deck 142 and side walls 144 are joined together to prevent materials from passing therebetween. Consequently, the solid waste material is contained in spaces, such as space 162, that are relatively simple to clean regularly and completely. As a further consequence, insect and rodent contact with the solid waste material prior to heating may be limited.

As noted above, the system 100 also includes at least one heating unit 130. In fact, as noted above, the system 100 includes four heating units 130. It will be recognized that the number of heating units 130 illustrated is non-limiting; a greater number or a lesser number of heating units 130 may be included.

As seen in Figs. 1A and 2A, the heating unit 130 may include a container (or drum) 170 with an opening 172 to receive solid waste material from the outlet 156 of the storage conveyor 120. The opening 172 may be in communication with an interior chamber 174. The heating unit 130 may also include a closure or door 176 moveable relative to the opening 172 to limit passage of solid waste material through the opening 172. According to the illustrated embodiment, the closure 176 may close the opening 172 so as to make the interior chamber 174 (and thus the container 170) airtight.

As seen in Fig. 2A, the heating unit 130 may also include a source of steam 178 coupled to the interior chamber 174 to provide steam to the interior chamber 174 to heat treat solid waste material disposed therein. As illustrated, a single source of steam 178 is coupled to the plurality of heating units 130 through a system of conduits or pipes 180. It will be recognized that the arrangement and number of sources of steam 178 relative to the arrangement and number of heating units 130 is a design choice influenced by a variety of factors.

The steam is released into the interior chamber 174 after first evacuating the air from the chamber 174. Pressures within the container 170 may exceed 50 psi during the heat treatment in the chamber 174. Furthermore, temperatures within the container 170 may exceed 500° Fahrenheit during this time. Consequently, the container 170 must be able to withstand such pressures and temperatures.

The container 170, or a portion thereof, may rotate about an axis. For example, a drum may be disposed within the chamber 174 for rotation about its longitudinal axis, which axis may be aligned with the longitudinal axis of the container 170. The interior surface of this drum may have angular or helical surfaces to provide proper agitation of the solid waste material in the chamber 174 during processing. For that matter, the rotation of the inner drum may facilitate movement of the solid waste materials into and out of the container 170 before and after processing. In this regard, the container 170 may have a structure and operation similar to the ROTOCLAVE® rotating autoclave system available from Tempico Inc. of Hammond, Louisiana.

To move the material from the storage conveyor 120 to the heating unit 130, a feed conveyor 190 may be provided. The feed conveyor 190 may be disposed between the outlet 156 of the storage conveyor 120 and the opening 172 of the container 170 to bridge a space 192 between the outlet 156 and opening 172. The space 192 may be provided, for example, to permit clearance for the door 174 to close over the opening 172. The space 192 may also permit clearance for treated solid waste materials to be ejected from the container 170 on to a different conveyor or transport system for transport away from the heating units 130.

The feed conveyor 190 may be similar in structure and operation to the storage conveyor 120, with certain differences to provide mobility. The feed conveyor 190 may include a trough 194 with a deck 196 and opposing side walls 198 disposed to either side of the deck 196. A moveable frame 200 is provided, the frame 200 having a first position (Fig. 2A) wherein the feed conveyor 190 bridges the space 192 between the outlet 156 and the opening 172 and a second position (Fig. 2B) wherein the feed conveyor 190 does not bridge the space 192 between the outlet 156 and the opening 172. In particular, the frame 200 may include a carrier or carriage 202 having pairs of opposing wheels 204, which wheels 204 may be mounted on rails 206 to guide the motion of the carrier 202. A plurality of resilient members 208 mount the trough 194 to the frame 200, and a vibration generator 210 is coupled to the trough 194 to move solid waste material disposed in the feed conveyor 190 between the outlet 156 of the storage conveyor 120 and the opening 174 of the heating unit 130.

In operation, the feed conveyor 190 would be maintained in the position illustrated in Fig. 2A to move the solid waste material from the storage conveyor 120 into the heating unit 130. Solid waste material may be moved from the storage conveyor 120 via feed conveyor 190 into the heating unit 130 at a rate such that 4500 cubic feet of solid waste material may be transferred from the conveyor 120 to the container 170 in six minutes. The feed conveyor 190 would then be moved (through the use of a hydraulic cylinder (not shown) arranged below the frame 200, for example) from the position illustrated in Fig. 2A to that illustrated Fig. 2B, and the door 176 may be closed to seal the container 170 of the heating unit 130. The feed conveyor 190 would then remain in this position until after the container 170 has ejected the treated solid waste material into a discharge conveyor 220 (see Figs. 1A and 2B).

The discharge conveyor 220 is the first in a series of conveyors that may define one or more transfer systems. In this regard, a transfer system may include a single conveyor, or a plurality of conveyors. A transfer system may couple various processing devices of the system 100 together through these conveyors alone, or in combination with other elements of the system 100. In fact, the processing devices may themselves define, in whole or in part, a transfer system. For that matter, the conveyors 120, 190 and the heating unit 130 may define one or more transfer systems depending on the perspective of the element of the system 100 under discussion.

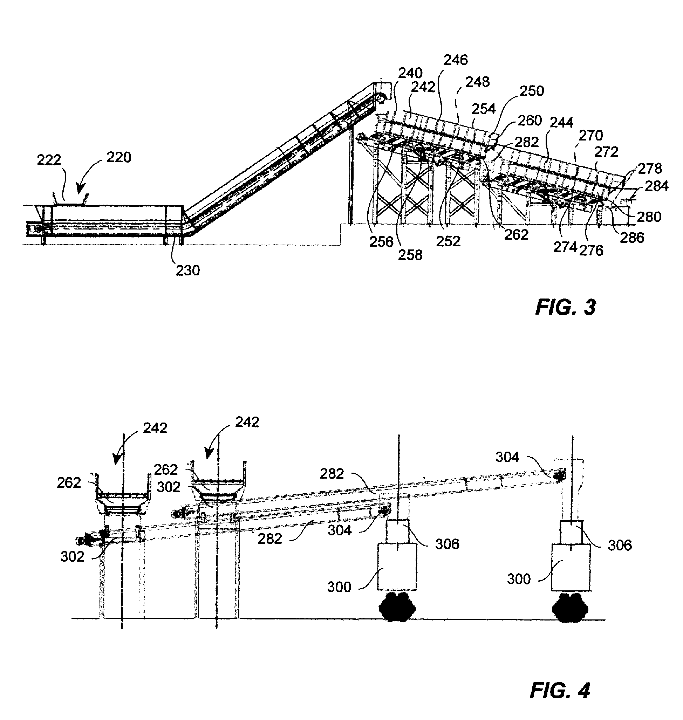

As illustrated in Fig. 1A, one discharge conveyor 220 is provided for two of the heating units 130. The number of discharge conveyors 220 may be determined according to the circumstances of a particular project. The discharge conveyors 220 may be vibratory conveyors, similar in structure and operation to the conveyors 120, 190 discussed above, or may be another type of conveyor. Given that the material being transported as this point is heat-treated (or simply "treated") solid waste material, use of non-vibratory conveyors does not present as many drawbacks as is the case for their use prior to the treatment of the solid waste material. In fact, as illustrated in Fig. 3, each discharge conveyor 220 may have an outlet 222 that is coupled to an inclined belt conveyor 230.

The inclined belt conveyor 230 transports the treated solid waste material to the inlet end 240 of a first of a pair of screening conveyors 242, 244 (see Figs. 1B and 3). Although two screening conveyors 242, 244 are illustrated, the number of screen conveyors according to other embodiments may be smaller or larger. The screening conveyors 242, 244 are coupled to the heating units 130 (via the transfer system including the conveyors 220, 230) to receive treated solid waste material from the heating units 130.

Each of the screening conveyors 242, 244 has a similar structure and operation. For example, referring to the screening conveyor 242, the conveyor 242 includes a screening conveyor trough 246 with a foraminous deck 248 and opposing side walls 250 disposed to either side of the deck 248. In fact, the sides 250 are coupled to the bottom 252 of the trough 246 so that material that passes through the deck 248 collects on the bottom 252 of the trough 242. The deck 248 may be defined by one or more conventional finger screens 254. A plurality of resilient members 256 supporting the trough 246 above a surface.

A vibration generator 258 is coupled to the trough 246 to move materials along the deck 248 and the bottom 252 of the trough 246. In particular, larger treated solid waste materials move along the deck 248 to an outlet 260. On the other hand, smaller treated solid waste materials pass through the deck 248, along the bottom 252 and to an outlet 262. In this fashion, the treated solid waste material is sorted according to physical size.

The materials passing through the outlet 260 are directed onto a deck 270 of the screening conveyor 244, which deck may be defined by one or more finger screens 272. Similar to the structure and operation of the conveyor 244, the larger treated solid waste materials move along the deck 270 while the smaller treated solid waste materials pass through the deck 270 and are collected on a bottom 274 of a trough 276. The materials moving along the deck 270 are directed to an outlet 278, while the materials moving along the bottom 272 are directed to an outlet 280.

It will be recognized that by selecting the size of the finger screens 254, 272 that define the foraminous decks 248, 270, three differently sized streams of treated solid waste material may be generated at outlets 262, 278, 280. Each of these streams is carried by a separate transfer system to a different part of the system 100 for further processing. Each of the transfer systems includes at least one belt conveyor 282, 284, 286, which conveyors are shown in part in Figs. 1B and 3, and separately in Figs. 4, 7, and 8.

The treated solid waste stream with the smallest relative physical size passes through outlet 262 and along conveyor 282 to a press 300; as illustrated, the treated solid waste material may pass to one of two presses 300 that are arranged to processes the material in parallel. The press 300 is the first of two different processing devices in the system 100 that may be used to remove moisture from the treated solid waste material to generate dried, treated solid waste material. As seen in Fig. 4, the conveyor 282 has an inlet 302 coupled to the outlet 262 of the screening conveyor 242 and an outlet 304 coupled to the inlet 306 of the press 300.

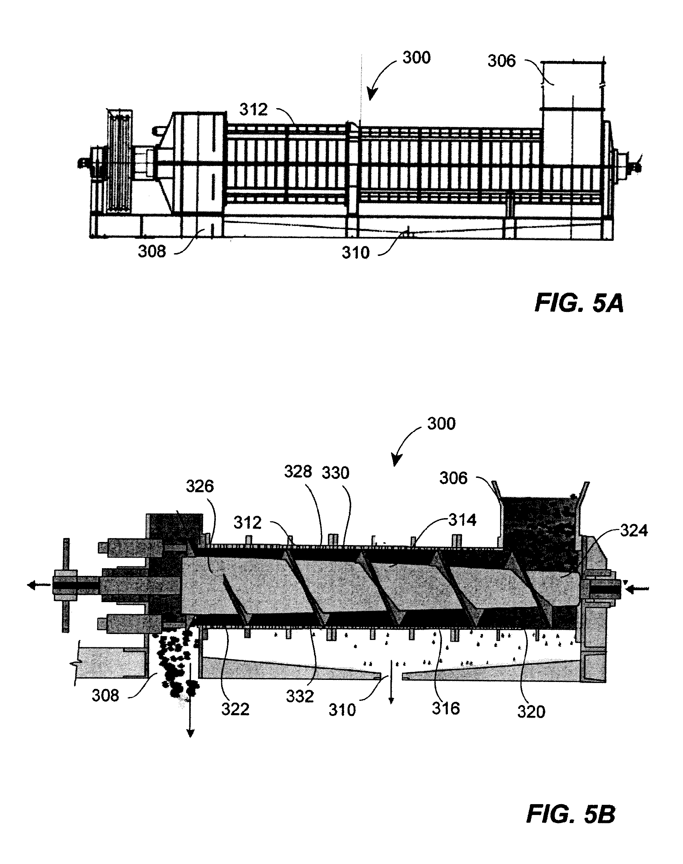

The press 300 may be a screw press, such as is illustrated in Figs. 5A and 5B. In addition to the inlet 306, the press 300 may have a first outlet 308 for passing the dried, treated solid waste material and a second outlet 310 for passing the filtrate that is removed from the treated solid waste material during handling. The inlet 306 and outlets 308, 310 may be defined by or attached to a housing 312 that receives the pressure cone 314 therein (see Fig. 5B). The moisture content of the treated solid waste material may be reduced from 70% to 45 % in the press 300.

The inner structure of the screw press is shown in greater detail in Fig. 5B. Solid waste material enters the housing 312 via the inlet 306. The solid waste material may enter with certain degree of moisture, shown to an exaggerated state in Fig. 5B. The housing 312 may include a screened section 316 that extends between the inlet 306 and the outlet 308, the screened section 316 having a first end 320 adjacent the inlet 306 and a second end 322 adjacent the outlet 308. The diameter of the screened section 316 between the first end 320 and the second end 322 is substantially constant. By contrast, the pressure cone 314 has an increasing diameter between a first end 324 and a second end 326, such that the clearance space between an inner surface 328 of the screened section 316 and an outer surface 330 of the pressure cone 314 decreases between the spaced ends. As a consequence, the moisture content of the material passing through the press 300 is ejected through the screened section 316, and passes out of the housing 312 via outlet 310. A vane or blade 332 may be attached to the pressure cone 314 to advance the solid waste material through the press 300 and eject the dried, treated solid waste material from the press 300.

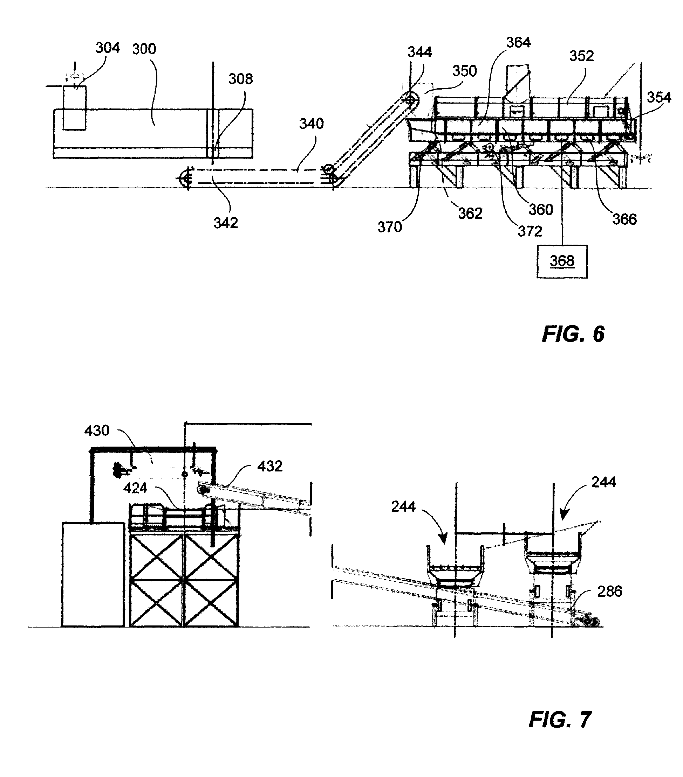

Coupled to the outlet 308 of the press 300 is a further transfer system, in this case including a belt conveyor 340 as shown in Fig. 6. In particular, the outlet 308 of the press 300 is coupled to an inlet 342 of the belt conveyor 340, and the outlet 344 of the belt conveyor 340 is coupled to an inlet 350 of a dryer 352.

The dryer 352 may be a conventional fluid bed dryer. The dryer 352 illustrated has the advantage of drying the treated solid waste material entering via the inlet 350 and moving the solid waste material from the inlet 350 to the outlet 354. In this regard, the dryer 352 may have a structure similar to the screening conveyors 342, 344, in that the dryer 352 may include a trough 360 with a deck 362 and opposing side walls 364 disposed on either side of the deck 362. An air plenum 366 is defined beneath the deck 362 to receive heated air therein from a source of heated air 368. The deck 362 has a plurality of openings therein to permit heated air from the plenum 366 to pass through the deck 362 and the material disposed above the deck 362. A plurality of resilient members 370 supports the trough 360 above a surface. A vibration generator 372 is coupled to the trough 360 to move materials along the deck 362.

The dried, treated solid waste material exiting dryers 352 via the respective outlets 354 is carried by a transfer system to a densifying and pelletizing apparatus 380. The transfer system may include several different devices for further sorting the dried treated solid waste material prior to its delivery to the densifying and pelletizing apparatus 380. The input of the transfer system is coupled to the outlet of the dryer 352 and the outlet of the transfer system is coupled to the inlet of the densifying and pelletizing apparatus 380.

The transfer system includes a first belt conveyor 390 is coupled to the outlets 354 of the dryers 352. An overhead belt magnet 392 is disposed at the output end of the belt conveyor 390 to attract and remove any metallic solid waste materials that may have been conveyed to this section of the system 100. The materials not removed by the belt magnet 392 are passed from the output end of the belt conveyor 390 to an input end of a second belt conveyor 394 with its output end coupled to the input end of a high-stroke feeder 396. The output end of the high-stroke feeder 396 may be coupled to the input end of a separation apparatus 398, such as a gear sorter. The output end of the separation apparatus 398 may be coupled in turn to further belt conveyor 400, which passes the dried, treated solid waste materials to dryer and cyclone separator 402, the output of which is coupled to the densifier and pelletizing apparatus 380. The pelletized, dried, treated solid waste material may then be transported to be used as fuel, for example.

Referring next to Figs. 1B, 7 and 8, the movement of the treated solid waste materials that exit the screening apparatuses 244 via the outlets 278, 280 is now discussed. Material exiting both outlets 278, 280 is transported via transfer systems to picking stations 420, 422. According to the illustrated embodiments, the transfer system from the outlet 278 to the picking station 420 includes belt conveyors 286, 424, while the transfer system from the outlet 280 to the picking station 422 includes belt conveyors 284, 426.

As illustrated in Figs. 1B and 7, an overhead belt magnet 430 may be arranged at an output end 432 of the belt conveyor 286 to separate any metallic materials that may remain in with the solid waste materials transferred along the belt conveyor 286. The non-metallic materials are passed along a series of individual stations via the conveyor 424, wherein various material are removed from the treated solid waste materials for separate processing, such as recycling. The materials that are not removed from the non-metallic materials at the picking stations 420 may be disposed of in a landfill, for example.

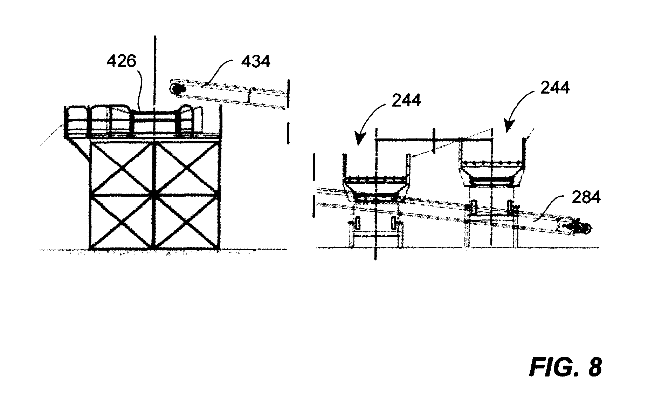

As illustrated in Figs. 1B and 8, an output end 434 of the belt conveyor 284 discharges at an input end of the conveyor 426. While no magnet is arranged at the output end 434 of the conveyor 284, a similar approach is followed at the picking station 422 as was followed at the picking station 420. Certain materials are removed from the treated solid waste materials for separate processing, while the remaining materials may be compacted in one of two compactors 436, 438 prior to transport to a landfill, for example.

It is believed that the present disclosure may have several benefits, one or more of which may be present in a particular embodiment according to the present disclosure.

## Claims

1. A vibratory system for processing solid waste material, comprising:
a storage conveyor including a trough with a deck and opposing side walls disposed to either side of the deck, a plurality of resilient members supporting the trough above a surface, and a vibration generator coupled to the trough to move solid waste material disposed in the trough along the deck to an outlet; and
a heating unit including a container with an opening to receive solid waste material from the outlet of the storage conveyor, the opening being in communication with an interior chamber, a closure moveable relative to the opening to limit passage of solid waste material through the opening, and a source of steam coupled to the interior chamber to provide steam to the interior chamber to heat solid waste material disposed therein.

2. The vibratory system for processing solid waste material according to claim 1, wherein the interior of the chamber is capable of withstanding pressures of at least 50 psi and temperatures of at least 500° Fahrenheit.

3. The vibratory system for processing solid waste material according to claim 1, comprising:
a feed conveyor disposable between the outlet of the storage conveyor and the opening of the container to bridge a space between the outlet and opening,
the feed conveyor including a feed conveyor trough with a deck and opposing side walls disposed to either side of the deck, a moveable frame having a first position wherein the feed conveyor bridges the space between the outlet and the opening and a second position wherein the feed conveyor does not bridge the space between the outlet and the opening, a plurality of resilient members mounting the trough to the frame, and a vibration generator coupled to the trough to move solid waste material disposed in the feed conveyor trough between the outlet and the opening.

4. The vibratory system for processing solid waste material according to claim 1, comprising:
at least one screening conveyor coupled to the opening to receive solid waste material from the heating unit,
the at least one screening conveyor including a screening conveyor trough with a foraminous deck and opposing side walls disposed to either side of the deck, a plurality of resilient members supporting the trough above a surface, and a vibration generator coupled to the screening conveyor trough to move larger solid waste materials along the deck to an outlet while smaller solid waste materials pass through the deck.

5. The vibratory system for processing solid waste material according to claim 4, comprising:
a transfer system with an inlet coupled to the outlet of the at least one screening conveyor to receive larger solid waste materials and an outlet;
at least one picking station coupled to the outlet of the transfer system.

6. The vibratory system for processing solid waste material according to claim 4, comprising:
a transfer system with an inlet coupled to the at least one screening conveyor to receive smaller solid waste materials that pass through the deck and an outlet;
at least one press with an inlet coupled to the outlet of the transfer system and an outlet.

7. The vibratory system for processing solid waste material according to claim 6, wherein the at least one press comprises a screw press.

8. The vibratory system for processing solid waste material according to claim 6, comprising a dryer with an inlet coupled to the outlet of the at least one press and an outlet.

9. The vibratory system for processing solid waste material according to claim 8, wherein the dryer comprises a fluid bed dryer.

10. The vibratory system for processing solid waste material according to claim 8, comprising a pelletizing system with a inlet coupled to the outlet of the dryer, the pelletizing system generating solid waste material pellets.

11. A method of processing solid waste material, comprising:
storing solid waste material in a storage conveyor, the conveyor including a trough with a deck and opposing side walls disposed to either side of the deck, a plurality of resilient members supporting the trough above a surface, and a vibration generator coupled to the trough to move solid waste material stored in the trough along the deck to an outlet;
activating the vibration generator to move the solid waste material along the deck to the outlet;
receiving the solid waste material in a container; and
supplying steam to the container to heat the solid waste material received therein to generate treated solid waste material.

12. The method of processing solid waste material according to claim 11, comprising:
supplying steam to the container so that pressure in the container is at least 50 psi and temperature in the container is at least 500° Fahrenheit.

13. The method of processing waste material according to claim 11, wherein receiving the solid waste material in a container comprises:
disposing the solid waste material in the container through an opening;
sealing the opening to make the container airtight.

14. The method of processing solid waste material according to claim 11, comprising:
removing the treated solid waste material from the container; and
sorting the treated solid waste material from the container according to physical size to generate a larger treated solid waste material stream and a smaller solid waste material stream.

15. The method of processing waste material according to claim 11, comprising:
removing the treated solid waste material from the container; and
sorting the treated solid waste material from the container using a magnet to generate a metallic treated solid waste material stream and a non-metallic solid waste material stream.

16. The method of processing waste material according to claim 11, comprising:
removing the treated solid waste material from the container; and
sorting the treated solid waste material from the container according to material type.

17. The method of processing waste material according to claim 11, comprising:
removing moisture from the treated solid waste material to generate dried, treated solid waste material.

18. The method of processing waste material according to claim 17, comprising:
applying pressure to the treated solid waste material to remove moisture therefrom.

19. The method of processing waste material according to claim 17, comprising:
passing heated air through the treated solid waste material to remove moisture therefrom.

20. The method of processing waste material according to claim 17, comprising:
pelletizing the dried, treated solid waste material.
